# EUROPEAN PATENT SPECIFICATION

(11) **EP 3 694 540 B1**
(45) Date of publication and mention of the grant of the patent: **20.11.2024**
(21) Application number: 18797284.9
(22) Date of filing: 11.10.2018
(51) Int. Cl.: A61K 38/48, C12N 9/52, C12N 15/00

(54) **BOTULINUM NEUROTOXIN A SUBTYPE 6 AND PHARMACOLOGICAL METHODS OF USE**
BOTULINUM NEUROTOXIN A SUBTYP 6 UND PHARMAKOLOGISCHE VERWENDUNGSVERFAHREN
SOUS-TYPE 6 DE LA NEUROTOXINE BOTULIQUE A ET PROCÉDÉS PHARMACOLOGIQUES D'UTILISATION

(30) Priority: 13.10.2017 US 201762572159 P
(43) Date of publication of application: 19.08.2020
(73) Proprietor: Wisconsin Alumni Research Foundation, Madison, WI 53726 (US)
(72) Inventor: JOHNSON, Eric, Madison WI 53703 (US); MORITZ, Molly, Madison WI 53726 (US); PELLETT, Sabine, Maison WI 53705 (US); BRADSHAW, Marite, Madison WI 53705 (US); TEPP, William, Stoughton WI 53589 (US)
(74) Representative: J A Kemp LLP
(86) International application number: PCT/US2018/055379
(87) International publication number: WO 2019/075182

(56) References cited:
- WO-A1-2015/166242
- LUQUEZ CAROLINA ET AL: "Neurotoxin Gene Clusters in Clostridium botulinum Type Ab Strains", APPLIED AND ENVIRONMENTAL MICROBIOLOGY, vol. 75, no. 19, October 2009 (2009-10-01), pages 6094 - 6101, XP002788141
- KULL SKADI ET AL: "Isolation and Functional Characterization of the Novel Clostridium botulinum Neurotoxin A8 Subtype", PLOS ONE, vol. 10, no. 2, February 2015 (2015-02-01), XP002788142

## Description

This invention was made with government support under NS083688 awarded by the National Institutes of Health. The government has certain rights in the invention.

### BACKGROUND OF THE INVENTION

The field of the invention is related to methods of obtaining purified preparations of *Clostridium botulinum* neurotoxin BoNT/A6. Also disclosed are compositions and methods of use.

Botulinum neurotoxins (BoNTs), the most poisonous substances known to man, are protein toxins produced by *Clostridium botulinum* and select strains of *Clostridium butyricum* and *Clostridium baratii* (Hill and Smith, 2013; Johnson and Montecucco, 2008). BoNTs are synthesized as 150 kDa dichain proteins made up of a 100 kDa heavy chain (HC) and a 50 kDa light chain (LC) linked by a disulfide bond. The HC is further divided into an N-terminal domain (H_{N}), which aids in translocation of the LC into the cell cytosol, and a C-terminal domain (Hc), which recognizes and bind to cell surface receptors on neuronal cells (Montal, 2010). Once inside the cell, the LC specifically cleaves a portion of a soluble N-ethylmaleimide sensitive-factor attachment protein receptors (SNARE) thereby inactivating neurotransmitter release (Montecucco and Schiavo, 1994; Schiavo et al., 1995). While BoNTs have the potential to cause serious illness, they are also able to be used effectively in the treatment of several neuromuscular disorders, comprising an over $3 billion and growing business.

BoNTs are divided into seven confirmed serotypes (A-G) which are further sub-categorized into subtypes based on variations in amino acid sequences (Gimenez and Gimenez, 1995; Hill et al., 2007; Hill and Smith, 2013; Montecucco, 2015; Smith et al., 2005). Despite the multitude of BoNT isotypes known today, only two are being employed as pharmaceuticals, namely BoNT/A1 and to a much lesser extent BoNT/B1. This is in part due to the relative lack of knowledge of pharmaceutical properties of most BoNT subtypes, which have been defined primarily based on their amino acid sequence and immunological properties.

Only relatively few BoNT subtypes have been characterized with regard to their biologic properties in the past few years. *In vitro* and *in vivo* investigations of BoNT/A subtypes 1-5 have revealed unique properties of some subtypes within a serotype, including potency, cell entry kinetics, and duration of action (Henkel et al., 2009; Tepp et al., 2012; Pier et al., 2011; Whitemarsh, 2013). In particular, BoNT/A2 was suggested as another subtype of choice for therapeutic treatment (Torii et al., 2010; Torii et al., 2014; Kaji, 2015) and is in clinical trials in Japan. It has been shown that BoNT/A2 enters cells faster and more efficiently than BoNT/A1 (Pier, 2010). Additional work has suggested that BoNT/A2 is more potent *in vivo* and remains localized within the injection site with less risk for side effects compared to BoNT/A1 (Torii et al., 2010; Torii et al., 2014; Kaji, 2015). In addition, recently detected BoNT/A subtypes A7 and A8 have been partially characterized *in vitro* (Morineaux et al., 2015).

However, for one BoNT/A subtype, BoNT/A6, little is known, in large part due to the fact that BoNT/A6 is produced in a dual toxin producing strain, which complicates its isolation and characterization. BoNT/A6 differs from other BoNT/A subtypes in the amino acid sequence by 14-4%, with the greatest similarity to A1 (95.7%) and A5 (95.9%), and greatest dissimilarity to A3 (86%) (Luquez et al., 2009; Morineaux et al., 2015). Interestingly, there is only 1 amino acid difference in the LC of A6 compared to A1 (T414A), with greater divergence in both the receptor binding domain and the translocation domain of the HC (Kull et al., 2015).

There is a need for other botulinum toxins that are characterized for use in formulations and botulinum toxin therapy.
The following documents are also mentioned:
- WO 2015/166242 which refers to manufacture of recombinant *Clostridium botulinum* neurotoxins;
- Luquez et al (2009) Applied and Environmental Microbiology, 75(19): 6094-6101 which refers to neurotoxin gene clusters in *Clostridium botulinum* Type Ab strains; and
- Kull *et al* (2015) *PLOS ONE,* 10(2) which refers to the isolation and functional characterization of the novel Clostridium botulinum A8 subtype

### SUMMARY OF THE INVENTION

The present invention provides method of obtaining a purified preparation of Clostridium botulinum neurotoxin BoNT/A6, wherein the method comprises:
(a) culturing a modified Clostridium botulinum strain CDC41370, wherein the stain is modified to remove the bont/B2 gene; and
(b) isolating the BoNT/A6 toxin from the strain, wherein the toxin is at least 90% pure BoNT/A6 toxin or toxin complex.

The present invention further provides a method of obtaining a purified preparation of Clostridium botulinum neurotoxin BoNT/A6, wherein the method comprises:
(a) culturing a Clostridium botulinum strain which only expresses BoNT/A6 toxin of the BoNT toxins; and
(b) isolating the BoNT/A6 toxin, wherein the toxin is at least 90% pure BoNT/A6 toxin or toxin complex.

This study describes the isolation and characterization of BoNT/A6 both *in vitro* and *in vivo.* The data described here show increased potency and faster cell entry kinetics of BoNT/A6 in cultured neuronal cell models compared to other BoNT/A subtypes including murine and rat primary neurons and human induced pluripotent stem cell (hiPSC) derived neurons. Mouse studies indicate a faster onset and an at least as long duration of action after local intramuscular injection compared to BoNT/A1, with a long duration of maximum local paralysis. Mouse studies also indicate less severe systemic symptoms after local injection of BoNT/A6 compared to other BoNT/A subtypes. These data indicate the potential for BoNT/A6 as a novel and improved biopharmaceutical.

Also disclosed is a method of treating a patient having a symptom that benefits from botulinum toxin therapy, comprising the step of treating the patient with an effective amount of a preparation comprising Clostridium botulinum neurotoxin BoNT/A6 or neurotoxin complex, wherein the preparation is at least 90% pure A6 toxin or toxin complex.

In some instances, the culture expresses a recombinant BoNT/A6 toxin. In other version, the culture is a modified version of a bacterial strain that natively expresses more than one toxin, including BoNT/A6.

Also disclosed is a preparation of Clostridium botulinum neurotozin BoNT/A6 which is at least 90% or at least 95% pure A6 toxin or toxin complex.

The foregoing and other aspects and advantages of the invention will appear from the following description. In the description, reference is made to the accompanying drawings which form a part hereof, and in which there are shown, by way of illustration, preferred embodiments of the invention. Such embodiments do not necessarily represent the full scope of the invention, however, and reference is made therefore to the claims and herein for interpreting the scope of the invention.

### BRIEF DESCRIPTION OF THE DRAWINGS

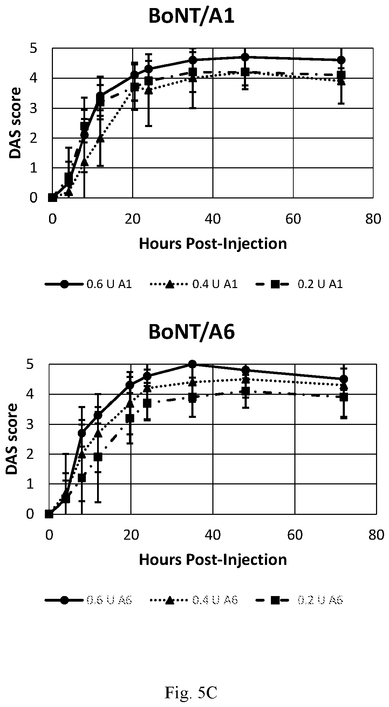

The patent or application file contains at least one drawing executed in color. Copies of this patent or patent application publication with color drawing(s) will be provided by the Office upon request and payment of the necessary fee.

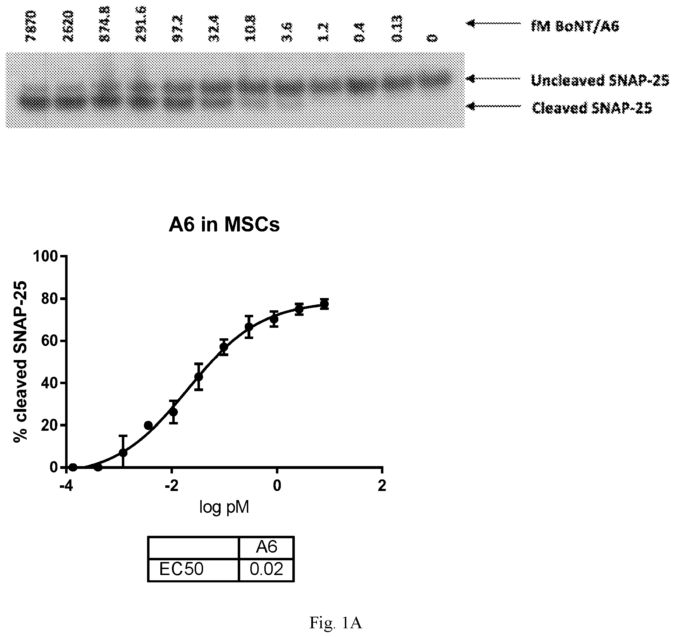

**Fig. 1A****. EC₅₀ of BoNT/A6 in primary MSC derived neurons.** Cells were exposed to serial dilutions of BoNT/A6 for 48 h. MSC cell lysates were analyzed by western blot and densitometry. A representative Western image and a graph depicting the average and standard deviation of triplicate samples is shown. The graphs were generated in PRISM 6 software and a nonlinear regression line was used to determine the EC₅₀ values. The EC50 was 0.02 pM. For comparison, the EC50 for BoNT/A1 is 0.15-0.2 pM.

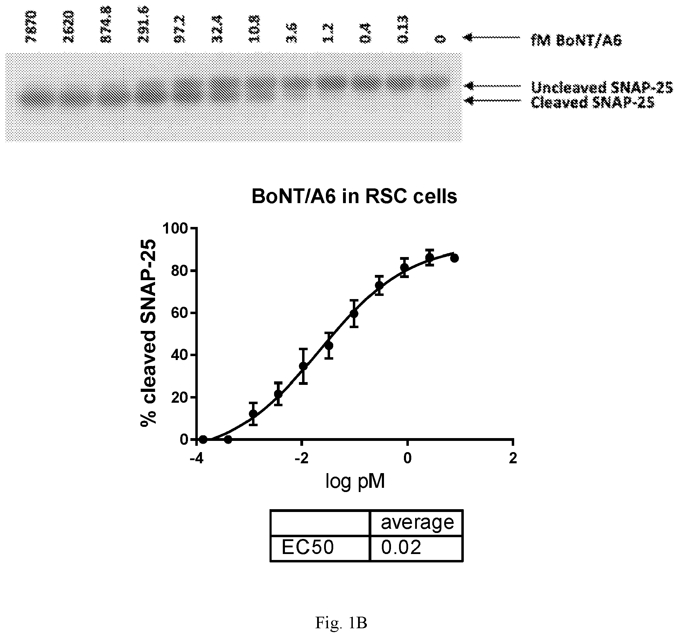

**Fig. 1B****. EC₅₀ of BoNT/A6 in RSCs derived neurons.** Cells were exposed to serial dilutions of BoNT/A6 for 48 h. RSC cell lysates were analyzed by western blot and densitometry. A representative Western image and a graph depicting the average and standard deviation of triplicate samples is shown. The graphs were generated in PRISM 6 software and a nonlinear regression line was used to determine the EC₅₀ values. The EC50 was 0.02 pM. For comparison, the EC50 for BoNT/A1 is 0.2 pM.

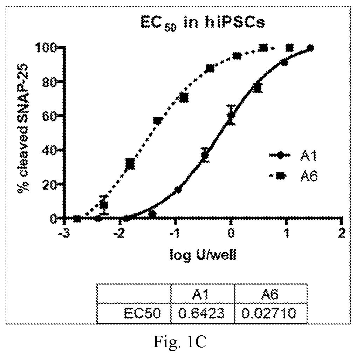

**Fig. 1C****. EC₅₀ of BoNT/A6 in hiPSC derived neurons.** Cells were exposed to serial dilutions of BoNT/A6 for 48 h. hiPSC cell lysates were analyzed by western blot and densitometry. A graph depicting the average and standard deviation of triplicate samples is shown. The graphs were generated in PRISM 6 software and a nonlinear regression line was used to determine the EC₅₀ values. The EC50 was 0.04 pM. For comparison, the EC50 for BoNT/A1 is 0.3 pM.

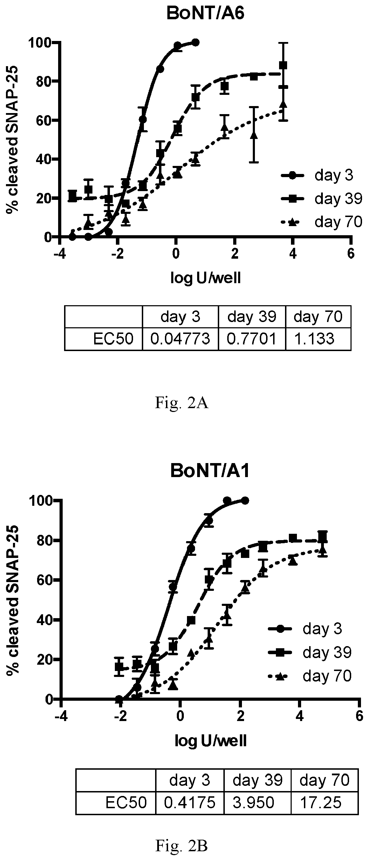

**Fig. 2A****. EC₅₀ and duration of action of BoNT/A6 in hiPSC derived neurons.** Human iPSC derived neurons were exposed to serial dilutions of BoNT/A6 for 72 h, followed by complete toxin removal. Cells were incubated in culture medium without toxin and were harvested on days 3, 39, and 70 after exposure. Graphs depicting the average and standard deviation of triplicate samples are shown. The graphs were generated in PRISM 6 software, and a nonlinear regression line was used to determine the EC₅₀ values.
**Fig. 2B****.** EC₅₀ **and duration of action of BoNT/A1 in hiPSC derived neurons.** Human iPSC derived neurons were exposed to serial dilutions of BoNT/A1 for 72 h, followed by complete toxin removal. Cells were incubated in culture medium without toxin and were harvested on days 3, 39, and 70 after exposure. Graphs depicting the average and standard deviation of triplicate samples are shown. The graphs were generated in PRISM 6 software, and a nonlinear regression line was used to determine the EC₅₀ values.

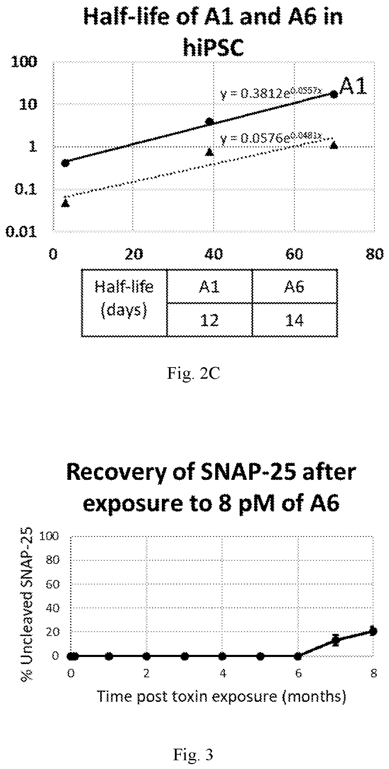

**Fig. 2C****. Half-life of BoNT/A1 and /A6 in hiPSC derived neurons.** The half-lives for A1 and A6 were estimated using the slopes of the regression line fit through the EC50 values over time using the formula t1/2 = LN(2)/slope. The half-lives were similar with ~12 and ~14 days for A1 and A6, respectively.
**Fig. 3****. Recovery of uncleaved SNAP-25 in RSCs.** RSCs were exposed to 8 pM of BoNT/A6. Cell lysates were analyzed for cleaved/uncleaved SNAP-25 by Western blot and densitometry through 8 months post toxin exposure. The Figure shows a long persistence of BoNT/A6 activity in primary rat spinal cord cells, similar or at least as long as previously observed for BoNT I A1.

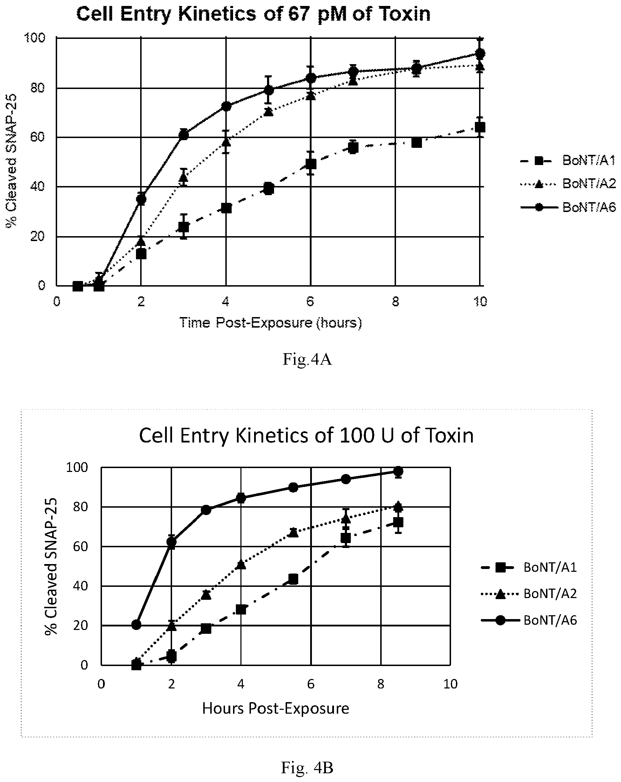

**Fig. 4A****. Cell entry kinetics of BoNT/A6 compared to A1 and A2.** iCell Neurons were exposed to the same amount of toxin and analyzed for SNAP-25 cleavage after exposure for the indicated time points. Cells were exposed to equal amounts of toxin in pM (A) (67 pM (500pg)) of BoNT/A1, /A2, and /A6.
**Fig. 4B****. Cell entry kinetics of BoNT/A6 compared to A1 and A2.** iCell Neurons were exposed to the same amount of toxin and analyzed for SNAP-25 cleavage after exposure for the indicated time points. Cells were exposed to equal amounts of toxin in Units of BoNT/A1, /A2, and /A6. The data show BoNT/A6 enters cells faster than BoNT/A1, and at least as fast as BoNT/A2.

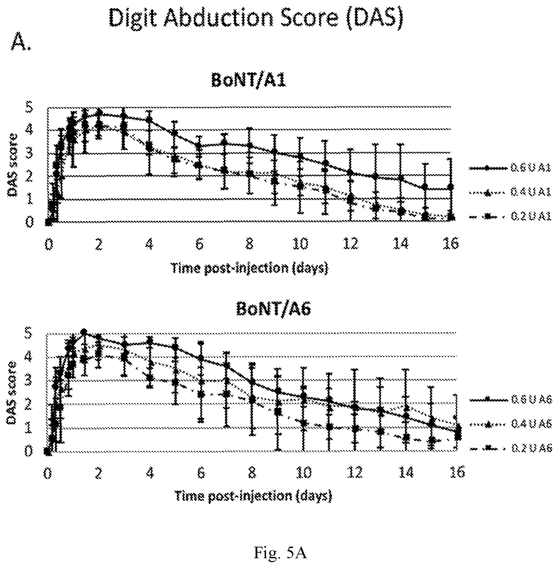

**Fig. 5A****. Onset and duration of action of BoNT/A6 and /A1 *in vivo.*** Average DAS scores of mice injected into the right gastrocnemius muscle with 0.6, 0.4, and 0.2 U of BoNT/A6 and BoNT/A1 are depicted graphically. n=5

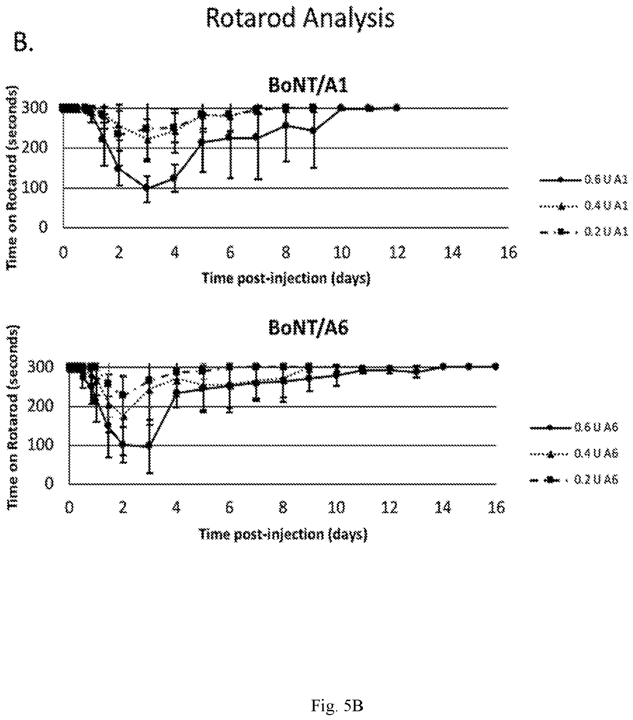

**Fig. 5B****. Onset and duration of action of BoNT/A6 and /A1 *in vivo.***
   Average Rotarod times of mice injected into the right gastrocnemius muscle with 0.6, 0.4, and 0.2 U of BoNT/A6 and BoNT/A1 are depicted graphically. n=5
**Fig. 5C****. Onset and duration of action of BoNT/A6 and /A1 *in vivo.*** Graphs depicting the DAS scores within the first 72 hours after injection were observed separately to compare the onset of maximum paralysis. n=5

### DETAILED DESCRIPTION OF THE INVENTION

Botulinum neurotoxin A subtype 6 (BoNT/A6) shows potential as an advantageous biopharmaceutical for therapeutic treatment of a wide variety of conditions as well as for aesthetic use compared to other BoNTs in commerce. Currently, only BoNT/A1 (Botox, Xeomin, Dysport) or to a lesser extent BoNT/B1 (Myobloc) are used as pharmaceuticals, comprising a $3 billion industry with strong economic growth. Studies on BoNT/A6 compared to other BoNT/A subtypes have shown that A6 appears to have advantages over treatments with A1 or A2.

The toxins have different structures and sequences. The amino acid sequence of A6 is different from that of A1 (4.3 % differences, mostly in the heavy chain). While the crystal structure of A1 has been determined, A6 has not been crystallized.

The sequence accession number in Genbank for A1 is A1 CAL82360.1 botulinum neurotoxin type A precursor [Clostridium botulinum A str. ATCC 3502, and for A6 is A6 ACW83608.1 botulinum neurotoxin type A [Clostridium botulinum].

Our results from human and rodent cell studies indicate that BoNT/A6 is over 10-fold more potent in cultured neurons, in particular in human neurons, compared to A1, has a faster onset of target (SNARE) cleavage in cultured neurons, and a long duration of action. *In vivo* mouse studies indicate a duration of action at least as long as A1 and A2, and less systemic distribution after local intramuscular injection compared to A1 and A2. Further, BoNT/A6 is at least as or more potent than A2.

Not to be bound by any theory, but treatments with A6 will be safer due to its ability to remain localized within the injection site and, therefore, decreasing the risk of side effects due to (systemic) spreading of the toxin. In addition, the greater potency and faster onset of action in neurons indicate that BoNT/A6 has potential as a more effective treatment, possibly requiring lower doses for the same pharmaceutical effect. As compared to BoNT/A1, BoNT/A6 will have the ability to relieve symptoms with a rapid onset of activity, a duration at least as long as A1 and A2, while lowering the risk of side effects by remaining more localized in injected tissues.

The present invention contemplates obtaining isolated preparations of the *Clostridium botulinum* neurotoxin BoNT/A6, wherein the preparations is at least 90% pure A6 toxin or toxin complex, and may include partial complex. In another embodiment, the preparation is at least 95% pure BoNT/A6 toxin or toxin complex. The present invention also contemplates obtaining isolated preparations of *Clostridium botulinum* neurotoxin BoNT/A6, wherein the preparations is at least 90% pure

The preparation obtained by the methods described herein include purified BoNT/A6 toxin complexes, including preparation comprising partial toxin complexes. In some embodiments, the preparations may further include stabilizers that are known to stabilize the BoNT proteins. Suitable stabilizers are known in the art, and include, but are not limited to, for example, human or bovine serum albumin, gelatin, recombinant albumin as described in US Publication US2005/0238663 among others.

In some embodiments, the Clostridium botulinum neurotoxin BoNT/A6 is at least about 10-fold more potent than BoNT / A1. In further embodiments, the Clostridium botulinum neurotoxin BoNT/A6 has an EC50 of about 20 to about 40 fM, for example about 30fM in rodent and human cell models. The BoNT/A6 has an EC50 which is similar to A2 in the same cell models. The EC50 of A1 is about 10-fold higher than for A6, i.e. 200-400 fM.

In some embodiments, the Clostridium botulinum neurotoxin BoNT/A6 has reduced systemic tissue toxin distribution compared to Clostridium botulinum neurotoxin BoNT/A1. In some embodiments, the BoNT/A6 neurotoxin is able to be localized and has reduced systemic tissue distribution when administered intramuscularly. Not to be bound by any theory, but this reduction in systemic tissue distribution allows for (1) higher dosages to be used as higher amounts remain concentrated at the area of contact and (2) more safety and reduction in unintentionally side effects as the neurotoxin remains near the site of administration. For example, larger amounts of BoNT/A6 may be injected as compared to BoNT/A1 which increases the effect and duration, which is dose dependent, and less systemic spread enables safe administration of these larger amounts of BoNT/A6.

In some instances, the same therapeutic effect as BoNT/A1 may be obtained by the use of a lesser amount of BoNT/A6.

Further, in one aspect, the higher potency of A6 in cultured neurons makes development of a cell-based assay for product release testing easier.

In another embodiment, the Clostridium botulinum neurotoxin BoNT/A6 has a specific activity of about 1-3 x 10⁷ LD50 units/mg, for example, about_1.7 x 10⁷ mouse LD50 Units/mg. The specific activity of the BoNT/A6 toxin or toxin complex is about the same as for BoNT/A1 in mice.

Also disclosed is a method of treating a patient having a symptom in need of botulinum toxin therapy, comprising the step of treating the patient with an effective amount of a preparation comprising Clostridium botulinum neurotoxin BoNT/A6. A preparation obtained by the methods of the invention is at least 90% pure BoNT/A6 toxin or toxin complex. In another embodiment, the preparation is at least 93% pure A6 toxin, alternatively at least 95% pure A6 toxin, alternatively at least 98% pure A6 toxin.

In some embodiments, the BoNT/A6 preparation has increased uptake in cells as compared to a preparation comprising BoNT/A2.

The terms "subject" and "patient" are used interchangeably and refer to any animal (e.g., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "treating" refers to reducing, eliminating, improving the condition of, or lessening the severity of any aspect of a symptom benefiting from botulinum toxin therapy in a patient. The present invention does not encompass methods of treatment practiced on the human or animal body and such methods are only disclosed for reference purposes. Also disclosed is a method of treating a patient having a symptom benefiting from botulinum toxic therapy. In such cases, the method can comprise treating the patient with an effective amount of a BoNT/A6 preparation disclosed herein whereby the symptom is reduced, eliminated or lessened.

A BoNT/A6 preparation as disclosed herein can be administered in a therapeutically effective amount. The terms "effective amount" or "therapeutically effective amount" refer to an amount sufficient to effect beneficial or desirable biological and/or clinical results. A BoNT/A6 preparation as disclosed herein can be administered in a therapeutically effective amount depending on the type of treatment necessary. Methods of determining suitable dosage or dosage ranges for individual treatment are known to those in the art. For methods provided herein, a BoNT/A6 preparation as disclosed herein can be administered by any means that achieves the intended purpose or is deemed appropriate by those skilled in the art. In an exemplary instance, a BoNT/A6 preparation is administered either as a single dose or, when appropriate, as continuous administration using, for instance, a mini pump system. In some cases, a BoNT/A6 preparation is provided as a liquid dosage form or as a lyophilized dosage form that is, for example, reconstituted prior to administration.

Not to be bound by any theory, formulations comprising BoNT/A6 toxin or toxin complex have a better safety profile due to the less systemic toxicity and thus can be used for any application in which botox therapy is used.

In one instance, a suitable dosage is from about 0.01 to 2,000 units, preferably 0.5 to 600 units are once administered intramuscularly for adults. One unit is defined herein as an amount of the toxin with which a half of mice die when administered intraperitoneally (1 LD₅₀). A total dose for patients is within a range of about 0.01 to 2,000 units. In one einstance, the suitable dosage is about 500- 1000 U.

The preparation of BoNT/A6 disclosed herein may suitably be used for treating a patient suffering from a disease with muscle over-activity, and in some instances, in a subject who has neutralizing antibodies to a type A1 botulinum toxin. The preparations described herein may be used to decrease local muscle over-activity. Suitable diseases include, but are not limited to, for example, cervical dystonia, blepharospasm, severe primary axillary hyperhidrosis, strabismus, achalasia, chronic focal neuropathies and migraine and other headache disorders, cosmetic issues, muscle spasms, upper motor neuron syndrome, sweating, neurological disorders treated with BoNT/A1, hemifacial spasm, spasmodic torticollis, post-stroke spasticity, cerebral palsy, spasmodic dysphonia, chronic pain such as lumbago, the stiffness in the shoulders, paresis occurring at the onset of Parkinson disease or multiple sclerosis, myofascial pain syndrome, spasm of the masticatory muscles, chronic anal fissure, overactive bladder, bruxism, facial myokymia, tic, local dystonia, or wrinkles, among others. Headaches, such as chronic migraines, can occur due to abnormal muscle overactivity in the neck and the shoulders and that muscular tension may abnormally be accelerated due to fatigue in the muscles ultimately induce chronic pains such as lumbago, pains at the neck or the back or the stiffness in the shoulders.

A disease with a muscle over-activity to be treated with the preparations disclosed herein is preferably a disease where rapid and long-lasting relaxation of a muscle over-activity is needed, i.e. a disease that needs to be treated with a preparation having immediate efficacy. Such a disease with a muscle over-activity includes one where a preparation is administered by adjusting a dose until an effective dose is determined where the treatment is done with cumulative efficacy. A systemic disease with a muscle over-activity includes systemic dystonia, systemic contracture, post-stroke spasticity, cerebral palsy, Parkinson disease and multiple sclerosis.

The preparation of the present disclosure may be administered at an effective amount. When administered to human, its preferable route of administration is topical administration or by injection, more preferably, intramuscular and subcutaneous administration. Timing and a dose of administration are not particularly limited and may vary depending upon severity of symptoms, age, sex, weight, site and route of administration.

In another instance, the treatment is a cosmetic treatment.

Suitable routes of administration for the preparations of BoNT/A6 toxin described herein include, but are not limited to application by direct injection or by topical application.

TA modified Clostridium botulinum stain comprising strain CDC41370 (available from the CDC) wherein the *bont*/*B2* gene is inactivated or knocked out can be used in the methods of producing the purified boNT/A6 toxin or toxin complex for methods described herein. Suitable methods of inactivation or knocking out the gene are known in the art, and include, CRISPR/Cas or pyre technology, or using the Clostron methodology is previously described for other strains in Pellett et al. 2016.

A method of obtaining a purified preparation of Clostridium botulinum neurotoxin BoNT/A6 is also provided, wherein the method comprises:
(a) culturing a modified Clostridium botulinum strain CDC41370, wherein the stain is modified to remove the bont/B2 gene; and
(b) isolating the BoNT/A6 toxin from the strain, wherein the toxin is at least 90% pure BoNT/A6 toxin or toxin complex.
The method is able to produce a preparation comprising at least 90% pure BoNT/A6, alternatively at least 95% pure BoNT/A6, alternatively at least 98% pure BoNT/A6.

The toxin of the present invention could be produced in various other ways. The strain described above and below is the only strain that is currently known to natively produce this toxin. However, the strain could be modified to completely knock out the second BoNT (B2) gene.

Also disclosed are kits able to be used in the methods described. For example, in one instance, the kit provides a preparation of BoNT/A6 which is about 90% pure BoNT/A6 toxin, and instructions for administration. Kits may also include, lyophilized BoNT/A6, and sterile pharmaceutically acceptable carrier in which to reconstitute the BoNT/A6 toxin before administration.

Suitable pharmaceutical carriers include, but are not limited to, for example, saline solution (e.g. 0.9% sodium chloride), phosphate buffer saline, lactated ringer's solution, and the like.

The invention will be more fully understood upon consideration of the following non-limiting examples.

### Examples

### Example 1: Isolation and characterization of BoNT/A6

This Example describes the isolation and characterization of BoNT/A6 both *in vitro* and *in vivo.* The data described here show increased potency and faster cell entry kinetics of BoNT/A6 in cultured neuronal cell models compared to other BoNT/A subtypes including murine and rat primary neurons and human induced pluripotent stem cell (hiPSC) derived neurons. Mouse studies indicate a faster onset and an at least as long duration of action after local intramuscular injection compared to BoNT/A1, with a long duration of maximum local paralysis. These data indicate the potential for BoNT/A6 as a novel and improved biopharmaceutical.

### Materials and Methods:

### Biosafety, biosecurity, and Ethics

The Johnson laboratory and personnel are registered with the Federal Select Agent Program for research involving botulinum neurotoxins (BoNTs) and BoNT-producing strains of clostridia. The research program, procedures, documentation, security, and facilities are closely monitored by the University of Wisconsin-Madison Biosecurity Task Force, the University of Wisconsin-Madison Office of Biological Safety, the University of Wisconsin-Madison Select Agent Program, and the Centers for Disease Control and Prevention (CDC) as part of the University of Wisconsin-Madison Select Agent Program. All personnel have undergone suitability assessments and completed rigorous and continuing biosafety training, including biosafety level 3 (BSL3) or BSL2 and select agent practices, before participating in laboratory studies involving BoNTs and neurotoxigenic C. *botulinum.* All animal experiments were approved by and conducted according to guidelines of the University of Wisconsin Animal Care and Use Committee.

### Botulinum Neurotoxins

BoNT/A1 and /A2 were purified from C. *botulinum* strains Hall *A-hyper* and Kyoto-F as previously described (Malizio et al., 2000; Tepp et al., 2012; Jacobson et al., 2011). BoNT/A6 was purified from a modified strain CDC41370, which expresses both BoNT/B2 and BoNT/A6. The BoNT/B2 gene was silenced using Clostron methodology as previously described for other strains (Pellett et al., 2016), resulting in strain CDC41370B2^{tox-}, which expressed exclusively BoNT/A6 at high levels. BoNT/A6 was then isolated from strain CDC41370B2^{tox-} using previously published methods described (Malizio et al. 2000; Tepp et al, 2012; Jacobson et al., 2011; Lin et al., 2010). The purity of the toxins was confirmed by spectroscopy and SDS-PAGE. The purified toxins were stored in 40% glycerol at -20°C until use. Specific activities of each subtype preparation were determined using an intraperitoneal mouse bioassay (MBA) as previously described. The specific activities of the toxins were 3.5 pg/LD50 (A1), 7.9 pg/LD50 (A2), and 5.95 pg/LD50 (A6).

### Western Blot Analysis

Western blot analyses were carried out as previously described (Pellett et al., 2010; Whitemarsh et al., 2012; Pellett et al., 2007). All cell lysates were separated on 12% Novex NUPAGE gels (Life Technologies) using MES running buffer, and transferred onto a 0.45 µm PVDF membrane (Millipore). Membranes were incubated in blocking buffer for 30 minutes and then in anti-SNAP-25 (Synaptic Systems) primary antibody solution overnight. After 5 5 min washes with washing buffer (KPL), the membranes were rinsed with ddH₂O and incubated in secondary anti-mouse antibody (KPL) solution for one hour. Five additional washes were done, membranes were rinsed with ddH₂O, and incubated in chemiluminescent substrate (Phosphaglo, KPL) for ~3 minutes. Images of bands on membrane were obtained and analyzed by densitometry with TotalLab Quant and PRISM 6 software.

### Primary rat (RSC) and mouse spinal cord (MSC) cells assay

Primary rat and mouse spinal cord cells were prepared as previously described (Pellett et al., 2007; Pellett et al., 2010). The cells were plated on a Techno Plastic Products (TPP) 96-well, flat-bottom plate coated with 0.01% poly-L-ornithine (Sigma) and 8.3µg/cm² matrigel (BD Biosciences). The cells were maintained in culture medium (CM) (Neurobasal medium supplemented with B27, glutamax, and penicillin/streptomycin [Invitrogen]) and allowed to mature for a minimum of 2 weeks. Cells were exposed to BoNT in 50 µl CM per well and incubated at 37° C in a 5% CO₂ humidified atmosphere for the indicated amount of time. Cells were lysed in 75 µl of 1X LDS lysis buffer and analyzed by Western blot.

To determine the effective concentration 50 (EC₅₀), MSCs and RSCs were exposed to 3-fold dilutions of BoNT in CM. Experiments were performed in at least triplicate, and a no-toxin control was included in each replicate. Toxin dilutions remained on cells for 48 hours at 37°C in a 5% CO₂ humidified atmosphere. Cells were then lysed and analyzed for the presence of cleaved and uncleaved SNAP-25 by western blot as previously described.

To determine the duration of action of BoNT/A6 in neuronal cells, RSCs were exposed to 8 pM of BoNT/A6, which is the minimum required to achieve 100% SNAP-25 cleavage in RSC cells, for 72 hours followed by extracellular toxin removal and further incubation in culture media without toxin as previously described for other BoNT/A subtypes (Whitemarsh et al., 2014). Cells were harvested at 3 days after initial toxin exposure and monthly thereafter until 8 months post-exposure. All time points were tested in quadruplet and included no toxin controls. Cleaved and uncleaved SNAP-25 were monitored over time by Western blot.

### iCell Neurons cell assay

iCell Neurons (Cellular Dynamics) were stored in liquid nitrogen until use. The cells were plated on a TPP 96-well, flat-bottom plate treated with 0.01% poly-L-ornithine and 8.3 µg/cm² matrigel-coated. The cells were maintained in iCell Neuron maintenance medium supplemented with iCell Neurons medium supplement (Cellular Dynamics) and matured for 9 days until toxin exposure.

To determine the EC₅₀ of BoNT/A6 in iCell Neurons, cells were exposed to fourfold dilutions of BoNT/A6 in culture medium for 72 h. To determine recovery of iCell Neurons from BoNT/A6 intoxication, cells were exposed to serial toxin dilutions for 72 hours, after which time all toxin was removed and cells were washed thoroughly to remove any extracellular toxin. Cells were harvested on days 3, 39, and 70 post-exposure.

To compare cell entry kinetics of BoNT/A1, /A2, and /A6, iCell Neurons were exposed to equal amounts, both in equal molarity (67 pM) and Units (100 U), of each toxin subtype. Cells were harvested at the indicated time points post toxin addition, and cell lysates were analyzed for SNAP-25 cleavage by Western blot. Entry kinetics were deduced based on the SNAP-25 cleavage determined by Western blot analysis and densitometry calculations of at least triplicate samples of each toxin at each time point.

### Intramuscular LD₅₀ in mice

Groups of 5 female ICR mice (Harlan) were injected with the indicated amounts of BoNT/A1, /A2, or /A6 in a 10 µl GelPhos buffer solution using a 0.3 cc insulin syringe into the right gastrocnemius muscle. Comparison groups of 5 mice received intraperitoneal injections of the same toxin dilutions in a 0.5 mL GelPhos solution. Mice were observed through 5-days post-injections, and any deaths were recorded. The LD₅₀ in pg was calculated for both the intramuscular (IM) and intraperitoneal (IP) injected mice using the Reed and Muench method (Reed and Muench, 1938). Based on the IP data and a Unit definition of 1 Unit = the amount of toxin required to result in death of 50% of mice after IP injection within 4 days, the specific activity of each toxin was determined in Units (IP LD₅₀) for the toxin dilutions used in this comparative assay. The IM LD₅₀ in Units was calculated based on the IP LD₅₀ values.

### Onset and duration of action of in vivo

Onset and duration of action was determined *in vivo* as previously described (Pellett et al. 2015). Using a 0.3 cc insulin syringe, groups of 5 female ICR mice were injected with the indicated amounts of BoNT/A6 in a 10 µl GelPhos solution into the right gastrocnemius. To determine the onset of local paralysis, the digit abduction score (DAS) was determined for the right hind limb of each mouse at several time points within the first 48 hours after injection and every 24 hours thereafter on a 1-5 scale. At the same time points, Rotarod analysis (MED-Associates) was performed on each mouse. Each mouse attempted to run for a total of 5 minutes while the Rotarod increased in speed from 4 to 40 rpm. Rotarod analysis was ceased for a group once all mice ran the full 5 minutes.

### Results:

### BoNT/A6 has a high potency in neuronal cells

Previous studies have described the potency of BoNT/A subtypes 1 through 5 in various cell models (Whitemarsh et al., 2013; Whitemarsh et al, 2014). Exposure of mouse and rat primary spinal cord cells (MSC and RSC cells) to serial dilutions of BoNT/A6 resulted in a SNAP-25 cleavage pattern similar to that previously observed for BoNT/A2, with 50% SNAP-25 cleavage occurring at ~0.03 U/50 µl/well (27 fM) of BoNT/A6 after 48 hours of toxin exposure (Figure 1). The EC₅₀ of BoNT/A6 was also determined in human iPSC derived neurons. As with other BoNT/A subtypes, exposure of hiPSC derived neurons resulted in a steep dose response curve extending from 0 to 100% cleavage within less than 3 logs for cells exposed to toxin for 3 days. The EC₅₀ was calculated to be -0.04 U/50 µl/well (32 fM) (Figure 1C), which is over 10-fold more sensitive than has been reported for BoNT/A1 (Whitemarsh et al., 2013). These data indicate that like BoNT/A2, BoNT/A6 is significantly more potent in cultured neurons than BoNT/A1, in particular in human neurons.

### Duration of action of BoNT/A6 in cultured neurons

A previous report has shown that the LCs of BoNT/A1, /A2, /A4, and /A5 remain active in cultured primary rat spinal cord neurons for over 10 months, and that the recovery rate for uncleaved SNAP-25 in the intoxicated cells is steady but very slow, reaching less than 50% after 10 months (Whitemarsh et al., 2014). To determine the duration of action of BoNT/A6 in cultured primary rodent neurons, RSC cells were exposed to enough BoNT/A6 to ensure complete SNAP-25 cleavage after 72 hours (8 pM). Cell lysates were analyzed at 3 days and each month post-exposure through 8 months for the appearance of uncleaved SNAP-25 to indicate neuronal cell recovery. At 7 months after initial toxin exposure, western blot analysis for the first time showed signs of slight uncleaved SNAP-25 recovery. After 8 months of recovery time, SNAP-25 cleavage in RSCs initially exposed to 8 pM remained high at 79% (Figure 3). Although signs of SNAP-25 recovery were not seen until later compared to previous work on BoNT/A subtypes 1-5, the slope of the line when signs of uncleaved SNAP-25 begin is similar to this previous data (Whitemarsh et al., 2014). The pattern of SNAP-25 recovery was similar for cells exposed to 4-fold more BoNT/A6 (32 pM) (data not shown).

Duration of action in cultured neurons was further investigated in human iPSC derived neurons by exposing the neurons to serial dilutions of either BoNT/A1 or BoNT/A6 for 72 h, followed by complete removal of extracellular toxin, and further incubation in culture media. Cells with each dilution series were harvested at days 3, 39, and 70 in triplicate, and the EC₅₀ for SNAP-25 cleavage was determined for each time point. For BoNT/A6, EC₅₀ values were ~0.04, 0.7, and 1 U/50 µl/well (32, 560, and 800 fM) at days 3, 39, and 70, respectively (Figure 2A). For BoNT/A1 EC₅₀ values were ~0.7, 6.3, and 28 U/50 µl/well (313, 2940, and 12,880 fM, respectively) at days 3, 39, and 70 (Figure 2B). The half-life of BoNT/A1 and /A6 in these hiPSC derived neurons as determined from the EC₅₀ values over time was similar for both BoNT/A1 and /A6, approximately 12 days and 14 days, respectively (Figure 2C). Taken together, these results indicate that BoNT/A6 has a long duration of action, similar to other BoNT/A subtypes.

### BoNT/A6 enters cells more efficiently than other BoNT/A subtypes

To examine the cell entry kinetics of BoNT/A6 compared to subtypes BoNT/A1 and /A2, hiPSC derived neurons were exposed to 67 pM of each of the toxins (Figure 4A). Cells were harvested at various time points through 10 hours post-exposure. Lysates were examined for the amount of cleaved and uncleaved SNAP-25. The onset of SNAP-25 cleavage occurred more rapidly in cells exposed to BoNT/A6 (Figure 4). These cells also experienced an average of 94 ± 5.3% SNAP-25 cleavage at the final 10-hour time point, while cells exposed to BoNT/A1 and /A2 reached approximately 64 ± 4% and 84 ± 1.2% cleavage, respectively. In this assay equimolar amounts of toxin were used where the three toxins had slightly varying specific activity (3.5 pg/LD₅₀ for A1, 7.9 pg/LD₅₀ for A2, and 6 pg/LD₅₀ for A6). A second assay exposing cells to the same biologic activity (number of Units) of each toxin (100 U /50 µl/well or 47 pM for A1, 105 pM for A2, and 80 pM for A6) resulted in similar data although the difference between BoNT/A2 and A6 was slightly diminished (Figure 4B). This indicates that BoNT/A6 has an earlier onset of activity in cultured neurons, similar or even earlier as has previously been described for BoNT/A2 (Pier et al.).

### BoNT/A6 IM LD₅₀ in mice

To determine if the faster cell entry of BoNT/A6 may lead to less toxin spread away from the injection site after local intramuscular injection, a relative IM LD₅₀ (relative to the IP LD₅₀ of the same toxin dilutions) in mice was determined for injections of BoNT/A1, /A2, and /A6 into the right gastrocnemius muscle. The IM LD₅₀ for BoNT/A6 was significantly higher than that of BoNT/A1 and similar to BoNT/A2, requiring 1.4 times the Units of toxin than BoNT/A1 (Table 1). This is in agreement with previous data comparing BoNT/A2 and BoNT/A1 (Torii et al, 2014).

**Table 1. IM LD₅₀ of BoNT/A1, /A2, and /A6. n=10**

| Intramuscular LD₅₀ values | | | |
|---|---|---|---|
| BoNT Subtype | IP LD50 (pg) | IM LD50 (pg) | IM LD50 (U) |
| A1 | 5.6 | 7.9 | 1.4 |
| A2 | 4.9 | 9.9 | 2.0 |
| A6 | 5.3 | 10.6 | 2.0 |

### Onset and duration of action of BoNT/A6 in vivo

To determine the onset and duration of action of BoNT/A6 compared to BoNT/A1, mice were injected with dilutions of either toxin ranging from 0.2 to 0.6 U into the right hind gastrocnemius muscle. DAS scores (to measure local paralysis) and Rotarod times (to measure overall motor-neuron deficiency) were recorded for each mouse at several time points throughout the first 48 hours and then once each day through 16-days post-injection. The injected doses of each toxin were confirmed by an IP LD₅₀ assay using the same dilutions (data not shown). As previously seen for other BoNTs, the DAS scores and Rotarod recovery times were both dose dependent (Figure 5). The peak DAS score appeared between 36 and 48-hours, and started to decrease at day 3-4, similar to previous reports for BoNT/A2 and slightly earlier than BoNT/A1 (Figure 5A,C). By day 16, the DAS score had dropped to ~1.5 for mice injected with the highest toxin dilution (Figure 5A) which is similar as previously observed with BoNT/A1, /A2, and /A5 (Pellett et al., 2015). Overall motor-neuron deficiency as measured by Rotarod was similar for both BoNT/A1 and /A6 (Figure 5B), and similar as has previously been observed for other BoNT/A subtypes (Pellett et al., 2015). These data indicate similar onset and duration of action of BoNT/A6 compared to BoNT/A1 for local paralysis in mice after local intramuscular injection, in addition to similar overall motor-neuron deficiency.

### Discussion:

BoNT/A subtype 6 was first described as a unique BoNT/A subtype, possibly derived from a recombination event of BoNT/A1 and A2 in 2009 (Luquez et al., 2009). The LC sequence of BoNT/A6 differs from that of A1 by only 1 amino acid residue (T414A), but the translocation and receptor binding domains differ by 4.5 and 9.5% respectively. The majority of the differences of the A6 translocation domain from A1 are also seen in A2, while most differences unique to A6 are seen in the receptor binding domain. BoNT/A6 is naturally expressed in a dual toxin producing strain, CDC41370, isolated from a foodborne botulism case in 1996 (Luquez et al., 2009). In addition to BoNT/A6, this strain also expressed BoNT/B2, complicating the isolation and characterization of the BoNT/A6 toxin. As has been previously done for the isolation of other BoNTs from bivalent strains (Pellett et al., 2016), strain CDC41370 was modified to eliminate expression of BoNT/B2 in this study. BoNT/A6 was expressed at high levels in the resulting single expressing strain CDC41370B2^{tox-}. BoNT/A6 was isolated, and biologic and pharmaceutical properties were examined *in vitro,* in cultured neurons, and in mice. Similar as has previously been shown for BoNT/A2, BoNT/A6 showed a higher potency than A1 in all tested cell models including primary rat and mouse spinal cord neurons and hiPSC derived neurons (Figures 1-2). These observations indicate that, like BoNT/A2, BoNT/A6 is about 10-fold more potent and has a faster onset of action than BoNT/A1 in cultured neurons including human neurons (Figures 2, 4). Compared to BoNT/A2, BoNT/A6 appeared to have about 2-fold greater potency in human neurons and a slightly faster onset of action, although examination of additional toxin preparations will be required to discern whether this observation reflects a statistically significant subtype specific difference. These data raise the question whether BoNT/A6 has the potential to remain more localized within the injection site after *in vivo* intramuscular injection, which would result in fewer side effects due to the toxin spreading from the injection site, and whether BoNT/A6 may have the potential to be effective sooner after injection compared to other currently available subtypes. In addition, more efficient and faster cell entry may lead to a requirement for lower doses of BoNT/A6 compared to BoNT/A1 to achieve the same local paralytic effect.

Since a previously analyzed BoNT/A subtype, BoNT/A3, has been shown to have a significantly shorter duration of action compared to BoNT/A1, duration of action of BoNT/A6 was examined both in cultured neurons and in mice as previously done for other BoNT/A subtypes. Primary rat spinal cord cells that had been exposed to the minimum amount of BoNT/A6 required to achieve 100% SNAP-25 cleavage were monitored for recovery of uncleaved SNAP-25 for up to 8 months. Mild recovery was clearly seen after 7 months post-exposure. After 8 months, over ~80% of the SNAP-25 remained cleaved (Figure 3). Previous similar studies for BoNT/A1-5 had shown first sign of recovery of uncleaved SNAP-25 at 3-4 months with about 50% of the SNAP-25 remaining cleaved at 9 months (Whitemarsh et al., 2014). Recovery of cultured neurons from BoNT/A1 and /A6 intoxication was also determined in human iPSC derived neurons (Figure 2). Interestingly, these neuronal cultures, which differ from the primary cultures in the species (human versus rat) as well as in cell composition (>98% pure forebrain-like neurons versus a mixture of spinal cord neurons and glial cells), recovered uncleaved SNAP-25 fully within less than 3 months. This enabled the estimation of the half-life of BoNT/A1 and /A6 in this human cell culture, with the data indicating similar half-life's of ~12 and ~14 days for A1 and A6, respectively (Figure 2C). These data indicate a duration of action of BoNT/A6 in RSCs at least as long as that of BoNT/A1. Similarly, *in vivo* studies using the mouse model indicated an at least as long duration of local paralysis after local intramuscular injection in the gastrocnemius muscle for BoNT/A6 as for BoNT/A1. Similarly, as has previously been observed for BoNT/A2, the onset of local paralysis was slightly earlier for BoNT/A6 compared to BoNT/A1 (Figure 5). These data indicate a similar duration of action and earlier onset of local paralysis of BoNT/A6 compared to BoNT/A1 in mice after local intramuscular injection.

To investigate the idea that BoNT/A6 may have less risk of side effects by reducing the amount of toxin that is spreading systemic, an LD₅₀ was determined for BoNT/A1, /A2, and /A6 for intramuscular injection into the right gastrocnemius muscle. This was based on the calculated LD₅₀ determined from a standard MBA with IP injections of the same toxin dilutions. IM injections of BoNT/A6 and BoNT/A2 required 2 times as much toxin to be lethal compared to the amount of toxin injected IP, while BoNT/A1 and only required 1.4 times as much toxin in an IM injection to be lethal compared to when injected IP, respectively (Table 1). Importantly, this was a comparative study determining IP and IM LD₅₀s in parallel for the same toxin dilutions of the three examined toxins, and the results indicate that intramuscular injection of BoNT/A6 results in less systemic toxin distribution than for BoNT/A1.

Based on our results, BoNT/A6 appears to have potential as a biopharmaceutical that may be more advantageous to BoNT/A1 and similar or better than BoNT/A2. Treatments with BoNT/A6 may relieve targeted symptoms faster with less risks for side effects without compromising the long-lasting effect. Through future work, BoNT/A6 may prove to be a safer and more effective option for treatments currently using other subtypes.

### References

Gimenez, D.F. and Gimenez, J.A. (1995) The typing of botulinal neurotoxins. Int. J. Food Microbiol. 27, 1-9. 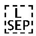
Henkel, J.S., Jacobson, M., Tepp, W., Pier, C., Johnson, E.A., Barbieri, J.T., 2009. Catalytic properties of botulinum neurotoxin subtypes A3 and A4 (dagger). Biochemistry 48, 2522.
Hill, K.K., Smith, T.J., 2013. Genetic diversity within Clostridium botulinum serotypes, botulinum neurotoxin gene clusters and toxin subtypes. Curr. Top. Microbiol. Immunol. 364, 1-20.
Hill, K.K., Smith, T.J., Helma, C.H., Ticknor, L.O., Foley, B.T., Svensson, R.T., Brown, J.L., Johnson, E.A., Smith, L.A., Okinaka, R.T., Jackson, P.J., Marks, J.D., 2007. Genetic diversity among botulinum neurotoxin-producing clostridial strains. J. Bacteriol. 189, 818-832.
Jacobson, M.J., Lin, G., Tepp, W., Dupuy, J., Stenmark, P., Stevens, R.C., Johnson, E.A., 2011. Purification, modeling and analysis of neurotoxin BoNT/A5 from Clostridium botulinum strain A661222. Appl. Environ. Microbiol. 77, 4217-4222.
Johnson, E.A., Montecucco, C. 2008. Chapter 11 botulism. In: Andrew, G.E. (Ed.), Handbook of Clinical Neurology, vol. 91. Elsevier, pp. 333-368.
Kaji, R. 2015. Clinical differences between A1 and A2 botulinum toxin subtypes. Toxicon. 107, 85-88.
Kull, S., Schulz, K.M., Weisemann, J., Kirchner, S., Schreiber, T., Bollenbach, A., Dabrowski, P.W., Nitsche, A., Kalb, S.R., Dorner, M.B., Barr, J.R., Rummel, A., Dorner, B.G., 2015. Isolation and functional characterization of the novel Clostridium botulinum neurotoxin A8 subtype, p e0116381. PLoS One 10. United States.
Lin, G., Tepp, W.H., Pier, C.L., Jacobson, M.J., Johnson, E.A., 2010. Expression of the Clostridium botulinum A2 neurotoxin gene cluster proteins and characterization of the A2 complex. Appl. Environ. Microbiol. 76, 40-47.
Luquez, C., Raphael, B.H., Maslanka, S.E., 2009. Neurotoxin gene clusters in Clostridium botulinum type Ab strains. Appl. Environ. Microbiol. 75, 6094-6101. United States.
Malizio, C.J., Goodnough, M.C., Johnson, E.A., 2000. Purification of Clostridium botulinum type A neurotoxin. Methods Mol. Biol. Clift. NJ. 145, 27-39.
Montal, M., 2010. Botulinum neurotoxin: a marvel of protein design. Annu. Rev. Biochem. 79, 591-617.
Montecucco, C., Rasotto, M.B., 2015. On botulinum neurotoxin variability. MBio 6.
Montecucco, C. and Schiavo, G. 1994. Mechanism of action of tetanus and botulinum neurotoxins. Mol. Microbiol. 13, 1-8.
Morineaux, V., Mazuet, C., Hilaire, D., Enche, J., Popoff, M.R. 2015. Characterization of botulinum neurotoxin type A subtypes by immunocapture enrichment and liquid chromatography-tandem mass spectrometry. Analytical and Bioanalytical Chemistry. 407, 5559-5570.
Pellett, S., Tepp, W.H., Clancy, C.M., Borodic, G.E. and Johnson, E.A. 2007. A neuronal cell-based botulinum neurotoxin assay for highly sensitive and specific detection of neutralizing serum antibodies. FEBS Lett. 581, 4803- 4808. 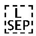
Pellett, S., Tepp, W.H., Bradshaw, M., Kalb, S.R., Dykes, J.K., Lin, G., Nawrocki, E.M., Pier, C.L., Barr, J.R., Maslanka, S.E., Johnson, E.A. 2016. Purification and Characterization of Botulinum Neurotoxin FA from a Genetically Modified Clostridium botulinum Strain. mSphere. 1(1):e00100-15.
Pellett, S., Tepp, W.H., Toth, S.I. and Johnson, E.A. 2010. Comparison of the primary rat spinal cord cell (RSC) assay and the mouse bioassay for botulinum neurotoxin type A potency determination. J. Pharmacol. Toxicol. Methods.
Pellett, S., Tepp, W.H., Whitemarsh, R.C.M., Bradshaw, M., Johnson, E.A. 2015. In vivo onset and duration of action varies for botulinum neurotoxin A subtypes 1-5. Toxicon. 107, 37-42.
Pier, C.L., Chen, C., Tepp, W.H., Lin, G., Janda, K.D., Barbieri, J.T., Pellett, S., Johnson, E.A., 2011. Botulinum neurotoxin subtype A2 enters neuronal cells faster than subtype A1. FEBS Lett. 585, 199-206. 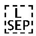
Reed LJ, Muench H. 1938. A simple method of estimating fifty per cent endpoints. Am J Hyg. 27,493-497.
Schiavo, G., Rossetto, O., Tonello, F. and Montecucco, C. (1995) Intracellular targets and metalloprotease activity of tetanus and botulism neurotoxins. Curr. Top. Microbiol. Immunol. 195, 257-274.
Smith, T.J., Lou, J., Geren, I.N., Forsyth, C.M., Tsai, R., Laporte, S.L., Tepp, W.H., Bradshaw, M., Johnson, E.A., Smith, L.A. and Marks, J.D. 2005. Sequence variation within botulinum neurotoxin serotypes impacts antibody binding and neutralization. Infect. Immun. 73, 5450-5457.
Tepp, W.H., Lin, G., Johnson, E.A., 2012. Purification and characterization of a novel subtype a3 botulinum neurotoxin. Appl. Environ. Microbiol. 78, 3108-3113.
Torii, Y., Goto, Y., Nakahira, S., Kozaki, S., Kaji, R., Ginnaga, A., 2014. Comparison of systemic toxicity between botulinum toxin subtypes A1 and A2 in mice and rats. Basic Clin. Pharmacol. Toxicol. 116, 524-528.
Torii, Y., Kiyota, N., Sugimoto, N., Mori, Y., Goto, Y., Harakawa, T., Nakahira, S., Kaji, R., Kozaki, S. and Ginnaga, A. 2010. Comparison of effects of botulinum toxin subtype A1 and A2 using twitch tension assay and rat grip strength test. Toxicon.
Whitemarsh RC, Strathman MJ, Chase LG, Stankewicz C, Tepp WH, Johnson EA, Pellett S. 2012. Novel application of human neurons derived from induced pluripotent stem cells for highly sensitive botulinum neu- rotoxin detection. Toxicol. Sci. 126:426 - 435.
Whitemarsh, R.C., Tepp, W.H., Bradshaw, M., Lin, G., Pier, C.L., Scherf, J.M., Johnson, E.A., Pellett, S., 2013. Characterization of botulinum neurotoxin A subtypes 1 through 5 by investigation of activities in mice. Neuronal. Cell Cult. Vitro. Infect. Immun. 81, 3894-3902.
Whitemarsh, R.C., Tepp, W.H., Johnson, E.A., Pellett, S., 2014. Persistence of botulinum neurotoxin A subtypes 1-5 in primary rat spinal cord cells. PLoS One 9, e90252.

The present invention is not intended to be limited to the foregoing examples, but encompasses all such modifications and variations as come within the scope of the appended claims.

## Claims

1. A method of obtaining a purified preparation of Clostridium botulinum neurotoxin BoNT/A6, wherein the method comprises:
(a) culturing a modified Clostridium botulinum strain CDC41370, wherein the stain is modified to remove the bont/B2 gene; and
(b) isolating the BoNT/A6 toxin from the strain, wherein the toxin is at least 90% pure BoNT/A6 toxin or toxin complex.

2. A method of obtaining a purified preparation of Clostridium botulinum neurotoxin BoNT/A6, wherein the method comprises:
(a) culturing a Clostridium botulinum strain which only expresses BoNT/A6 toxin of the BoNT toxins; and
(b) isolating the BoNT/A6 toxin, wherein the toxin is at least 90% pure BoNT/A6 toxin or toxin complex.

3. The method of claim 1 or 2, wherein the purified A6 toxin or toxin complex is in a form suitable for administration to a patient.

4. The method of claim 1 or 2, further comprising step (c) providing the A6 toxin or toxin complex in a single dose form.

5. The method of claim 1 or 2, further comprising step (c) providing the A6 toxin or toxin complex in a liquid dosage form or as a lyophilized dosage form.

## Patentansprüche

1. Verfahren zum Erhalten einer gereinigten Zubereitung von Clostridium botulinum Neurotoxin BoNT/A6, wobei das Verfahren umfasst:
(a) Züchten eines modifizierten Stammes von Clostridium botulinum CDC41370, wobei der Stamm modifiziert ist, um das Bont/B2-Gen zu entfernen; und
(b) Isolieren des BoNT/A6-Toxins aus dem Stamm, wobei das Toxin zu wenigstens 90 % reines BoNT/A6-Toxin oder ein Toxin-Komplex ist.

2. Verfahren zum Erhalten einer gereinigten Zubereitung von Clostridium botulinum Neurotoxin BoNT/A6, wobei das Verfahren umfasst:
(a) Züchten eines Stammes von Clostridium botulinum, der nur BoNT/A6-Toxin der BoNT-Toxine exprimiert; und
(b) Isolieren des BoNT/A6-Toxins, wobei das Toxin zu wenigstens 90 % reines BoNT/A6-Toxin oder ein Toxin-Komplex ist.

3. Verfahren nach Anspruch 1 oder 2, wobei das gereinigte A6-Toxin oder der Toxin-Komplex in einer Form vorliegt, die für die Verabreichung an einen Patienten geeignet ist.

4. Verfahren nach Anspruch 1 oder 2, das ferner den Schritt (c) des Bereitstellens des A6-Toxins oder des Toxin-Komplexes in einer Einzeldosisform umfasst.

5. Verfahren nach Anspruch 1 oder 2, das ferner den Schritt (c) des Bereitstellens des A6-Toxins oder des Toxin-Komplexes in einer flüssigen Dosierungsform oder als eine lyophilisierte Dosierungsform umfasst.

## Revendications

1. Procédé permettant d'obtenir une préparation purifiée de la neurotoxine Clostridium botulinum BoNT/A6, dans lequel le procédé comprend :
(a) la culture d'une souche modifiée de Clostridium botulinum CDC41370, dans laquelle la souche est modifiée pour supprimer le gène bont/B2 ; et
(b) l'isolement de la toxine BoNT/A6 à partir de la souche, dans lequel la toxine est une toxine ou un complexe de toxine BoNT/A6 d'une pureté d'au moins 90 %.

2. Procédé permettant d'obtenir une préparation purifiée de la neurotoxine Clostridium botulinum BoNT/A6, dans lequel le procédé comprend :
(a) la culture d'une souche de Clostridium botulinum qui n'exprime que la toxine BoNT/A6 des toxines BoNT ; et
(b) l'isolement de la toxine BoNT/A6, dans lequel la toxine est une toxine ou un complexe de toxine BoNT/A6 d'une pureté d'au moins 90 %.

3. Procédé selon la revendication 1 ou 2, dans lequel la toxine ou le complexe de toxine A6 purifié se présente sous une forme adaptée à une administration à un patient.

4. Procédé selon la revendication 1 ou 2, comprenant en outre l'étape (c) consistant à fournir la toxine ou le complexe de toxine A6 sous forme de dose unitaire.

5. Procédé selon la revendication 1 ou 2, comprenant en outre l'étape (c) consistant à fournir la toxine ou le complexe de toxine A6 sous forme posologique liquide ou sous forme posologique lyophilisée.
